# EUROPEAN PATENT APPLICATION

(11) **EP 2 581 064 A1**
(43) Date of publication of application: **17.04.2013**
(21) Application number: 12195744.3
(22) Date of filing: 05.05.2003
(51) Int. Cl.: A61F 2/90, A61F 2/91, A61F 2/06

(54) **Shape memory polymer stent**

(30) Priority: 14.05.2002 US 145387
(62) Divisional of application: 03741776.3
(71) Applicant: Micrus Endovascular Corporation, San Jose, CA 95131 (US)
(72) Inventor: Debeer, Niholas C., Montana, CA 94037 (US); Kurz, Daniel R., Monterey, CA 93940 (US); Ferrara, David A., Manhattan Beach, CA 90266 (US)
(74) Representative: McLeish, Nicholas Alistair Maxwell

(57) **Abstract**

Described herein is an intravascular flow modifier that comprises a plurality of annular support members and a plurality of cross-struts. The plurality of annular support members are interwoven with the plurality of cross-struts to form a tubular framework. The annular support members and cross-struts are formed of a shape memory polymer which has shape memory properties and a glass transition temperature that is above body temperature. The tubular framework has a compressed configuration at a temperature below body temperature and an expanded configuration at a temperature above body temperature. In the expanded configuration the tubular framework has an enlarged inner and outer diameter when it is heated above the glass transition temperature.

## Description

### BACKGROUND OF THE INVENTION

### Field of the Invention:

This invention relates generally to implantable devices for interventional therapeutic treatment or vascular surgery, and more particularly concerns a shape memory polymer stent.

### Description of Related Art:

The art and science of interventional therapy and surgery has continually progressed towards treatment of internal defects and diseases by use of ever smaller incisions or access through the vasculature or body openings in order to reduce the trauma to tissue surrounding the treatment site. One important aspect of such treatments involves the use of catheters to place therapeutic devices at a treatment site by access through the vasculature. Examples of such procedures include transluminal angioplasty, placement of stents to reinforce the walls of a blood vessel or the like and the use of vasoocclusive devices to treat defects in the vasculature. There is a constant drive by those practicing in the art to develop new and more capable systems for such applications. When coupled with developments in biological treatment capabilities, there is an expanding need for technologies that enhance the performance of interventional therapeutic devices and systems.

One specific field of interventional therapy that has been able to advantageously use recent developments in technology is the treatment of neurovascular defects. More specifically, as smaller and more capable structures and materials have been developed, treatment of vascular defects in the human brain which were previously untreatable or represented unacceptable risks via conventional surgery have become amenable to treatment

Stents are typically implanted within a vessel in a contracted state and expanded when in place in the vessel in order to maintain patency of the vessel, and such stents are typically implanted by mounting the stent on a balloon portion of a balloon catheter, positioning the stent in a body lumen, and expanding the stent to an expanded state by inflating the balloon. The balloon is then deflated and removed, leaving the stent in place. However, the placement, inflation and deflation of a balloon catheter is a complicated procedure that involves additional risks beyond the implantation of the stent, so that it would be desirable to provide a stent that can be more simply placed in the site to be treated in a compressed state, and expanded to leave the stent in place.

A number of stents formed from polymeric memory materials are known that transform from a compressed configuration to an expanded configuration. One such conventional stent is known, for example, that provides a casing formed from a memory elastomer such as polyurethane, and a support structure that can be manufactured by braiding individual threads formed of a temperature-sensitive polyurethane that is hard below 25°C and that softens above 35°C, so that at a temperature slightly below body temperature, the stent changes from a pressed configuration to an expanded configuration.

However, stents formed of shape memory polymeric materials typically do not provide adequate structural and mechanical radial strength requirements for a stent. Stents are therefore commonly provided with a metallic structure to provide the strength required to function as a stent. It would therefore be desirable to provide a shape memory polymer stent having a configuration that would provide adequate structural and mechanical radial strength for a stent, and that can be deployed without requiring inflation and deflation of a balloon catheter, by pushing the stent in a compressed state for deployment at the site to be treated, where the stent can be expanded to leave the stent in place. It would also be desirable to provide a stent formed of a shape memory polymer that has a glass transition temperature (T_{g}) above body temperature to allow for a controlled transition from a compressed configuration to an expanded configuration when exposed to body temperature, by controlled heating of the stent. The present invention meets these and other needs.

### SUMMARY OF THE INVENTION

Briefly, and in general terms, the present invention provides for a stent that is made from a polymer having shape memory properties so as to be self expanding, and that is therefore atraumatic to vasculature lumens of the body. The stent can be used within the vascular system as a means of preventing restenosis of vessels or as an intravascular flow modifier that is useful in treating cerebral or abdominal aortic aneurysms.

The invention accordingly provides, in a first embodiment, for a shape memory polymer stent, comprising an extruded tube having a truss-like design, and formed from a polymer having shape memory properties. In one presently preferred aspect, the polymer can be a polyurethane that can be compressed from an originally expanded configuration with a predetermined shape to have a reduced diameter to fit into a catheter or delivery system, and that can return to its predetermined shape and original expanded diameter after heating of the stent above its glass transition temperature. The stent can, for example, be formed as an extruded tube, and processed to remove segments yielding a truss-like design for improved radial strength.

In a second embodiment, the invention provides for a shape memory polymer stent, comprising a tube woven from extruded strands of a polymer having shape memory properties. In one presently preferred aspect, the polymer can be a polyurethane that can be compressed from an originally expanded configuration with a predetermined shape to have a reduced diameter to fit into a catheter or delivery system, and that can return to its predetermined shape and original expanded diameter after heating of the stent above its glass transition temperature.

In each of the foregoing embodiments, after formation of the stent in its expanded configuration with a predetermined shape, by heating the stent above its glass transition temperature (T_{g}), the stent of shape memory material transitions into the rubbery state and can be compressed to be axially stressed in the distal direction to have a reduced diameter and increased length. In one presently preferred embodiment, the stent of the invention can be compressed over a mounting portion of a pusher catheter for deployment within the vasculature. In a preferred aspect, the outer diameter of the pusher member on either side of the stent is smaller than the inner diameter of the stent in its expanded configuration but greater than the inner diameter of the stent in its compressed configuration, while the outer diameter of the mounting portion of the pusher member over which the stent is placed has a reduced diameter that is less than or equal to the inner diameter of the stent in its compressed configuration.

In the elongated state, the stent can fit within a catheter or other delivery system for delivery through the vasculature. Because the stent is formed from a shape memory material, it will return to its original shape and dimensions to relieve the external stress of compression, if allowed to remain above T_{g}. However, before the stent can recover its original shape and dimensions, it can be fixed in the compressed, elongated configuration and mounted over the mounting portion of the pusher catheter by lowering the temperature of the material below T_{g}. The stent can then be inserted into the vasculature and maneuvered into a desired location mounted on the pusher catheter, and heat can be transferred to the stent from the pusher catheter, such as by transmission of light energy, through a heat pipe, by conducting electricity through electrical resistance, transmission of radio-frequency electro-magnetic waves or ultra-sonic waves, or other means. The heat transfer causes the temperature of the stent to once again rise above T_{g} and causes the stent to transition back into the rubbery state to radially expand and axially retract to its original shape and dimensions, deploying the stent in the vasculature and allowing the pusher catheter to be retracted from the vasculature.

These and other aspects and advantages of the invention will become apparent from the following detailed description and the accompanying drawings, which illustrate by way of example the features of the invention.

Thus in summary, according to a first aspect of the present invention there is provided a shape memory polymer stent having a tubular framework, comprising: a plurality of annular support members; and a plurality of cross-struts intersecting said plurality of annular support members.

Advantageously the tubular framework is formed from an extruded tube having a surface defining said plurality of annular support members, said plurality of cross-struts, and a plurality of openings between said plurality of annular support members and said plurality of cross-struts.

Advantageously the plurality of cross-struts extend at an oblique angle relative to said plurality of annular support members.

Advantageously the extruded tube is formed of a polymer having shape memory properties. Preferably the polymer is polyurethane. Also preferably the polymer has a glass transition temperature and a predetermined shape having an expanded diameter after heating above the glass transition temperature, and a reduced diameter to fit into a catheter or delivery system.

Advantageously the tubular framework is formed from woven strands of said plurality of annular support members and said plurality of cross-struts.

According to a second aspect of the present invention there is provided a shape memory polymer stent having a tubular framework, comprising: a plurality of annular support members; and a plurality of cross-struts, said plurality of annular support members being interwoven with said plurality of cross-struts in a tubular shape.

Advantageously the tubular framework is formed from woven strands of said plurality of annular support members and said plurality of cross-struts.

Advantageously the plurality of cross-struts extend longitudinally.

Advantageously the strands are formed of a polymer having shape memory properties. Preferably the polymer is polyurethane. Also preferably the polymer has a glass transition temperature and a predetermined shape having an expanded diameter after heating above the glass transition temperature, and a reduced diameter to fit into a catheter or delivery system.

According to a third aspect of the present invention there is provided an intravascular flow modifier having a tubular framework that is useful in treating cerebral or abdominal aortic aneurysms, the intravascular flow modifier comprising: a plurality of annular support members; and a plurality of cross-struts intersecting said plurality of annular support members.

Advantageously the tubular framework is formed from an extruded tube having a surface defining said plurality of annular support members, said plurality of cross-struts, and a plurality of openings between said plurality of annular support members and said plurality of cross-struts.

Advantageously the plurality of cross-struts extend at an oblique angle relative to said plurality of annular support members.

Advantageously the extruded tube is formed of a polymer having shape memory properties. Preferably the polymer is polyurethane. Also preferably the polymer has a glass transition temperature and a predetermined shape having an expanded diameter after heating above the glass transition temperature, and a reduced diameter to fit into a catheter or delivery system.

Advantageously the tubular framework is formed from woven strands of said plurality of annular support members and said plurality of cross-struts.

According to a fourth aspect of the present invention there is provided an intravascular flow modifier having a tubular framework, comprising: a plurality of annular support members; and a plurality of cross-struts, said plurality of annular support members being interwoven with said plurality of cross-struts in a tubular shape.

Advantageously the tubular framework is formed from woven strands of said plurality of annular support members and said plurality of cross-struts.

Advantageously the plurality of cross-struts extend longitudinally.

Advantageously the strands are formed of a polymer having shape memory properties. Preferably the polymer is polyurethane. Also preferably the polymer has a glass transition temperature and a predetermined shape having an expanded diameter after heating above the glass transition temperature, and a reduced diameter to fit into a catheter or delivery system.

According to a fifth aspect of the present invention there is provided a method of introducing a shape memory polymer stent into a target site of a blood vessel to be treated, the method comprising the steps of : providing an elongated pusher member having distal and proximal ends, the elongated pusher member having a principal outer diameter over the majority of the length of the elongated pusher member, and elongated pusher member having a distal seating region having an outer diameter that is less than the principal outer diameter; providing a tubular shape memory polymer stent having a compressed configuration at a temperature below body temperature and an enlarged configuration at a temperature above body temperature, the tubular shape memory polymer stent in the expanded configuration having an enlarged inner and outer diameter when heated above a glass transition temperature that is above body temperature, the enlarged inner diameter being greater than the principal outer diameter of the elongated pusher member, and the tubular shape memory polymer stent in the compressed configuration having a reduced inner and outer diameter smaller than the enlarged inner and outer diameter of the tubular shape memory polymer stent, respectively, the reduced inner diameter being larger than the outer diameter of the seating region of the elongated pusher member and smaller than the principal outer diameter of the elongated pusher member; mounting the tubular shape memory polymer stent in the compressed configuration over the seating region of the elongated pusher member so as to trap the tubular shape memory polymer stent on the seating region of the elongated pusher member; introducing the distal seating portion of the elongated pusher member and tubular shape memory polymer stent mounted thereon into a lumen of a catheter; positioning the catheter within the vasculature so that the distal opening of the catheter is proximal to the target site of the blood vessel to be treated; pushing the distal seating portion of the elongated pusher member carrying the tubular shape memory polymer stent out of the distal opening of the catheter to the target site of the blood vessel to be treated; heating the tubular shape memory polymer stent to cause the tubular shape memory polymer stent to transition to the expanded configuration, thereby deploying the tubular shape memory polymer stent within the aneurysm and at least partially occluding the opening between the aneurysm and the parent blood vessel.

Advantageously the step of heating the tubular shape memory polymer stent comprises causing energy to be transmitted through the elongated pusher member to release the connection between the pusher member and the tubular shape memory polymer stent.

Advantageously the elongated pusher member is a fiber optic, and the step of heating comprises conducting light energy to said seating region of said elongated pusher member to heat the tubular shape memory polymer stent. Alternatively the elongated pusher member is a heat pipe, and the step of heating comprises conducting heat along said elongated pusher member to said seating region of said elongated pusher member to heat the tubular shape memory polymer stent. Alternatively the step of heating comprises heating said tubular shape memory polymer stent by RF energy.

### BRIEF DESCRIPTION OF THE DRAWINGS

FIGURE 1 is a plan view of a first embodiment of the stent of the invention formed from an extruded tube and processed to remove segments yielding a truss-like design, in an original expanded configuration in a predetermined shape.
Fig. 2 is an end view of the stent of Fig. 1.
Fig. 3 is a perspective view of the stent of Fig. 1.
Fig. 4 is a perspective view of the stent of Fig. 1 in a compressed, elongated configuration.
Fig. 5 is a plan view of a second embodiment of the stent of the invention woven from extruded strands, in an original expanded configuration in a predetermined shape.
Fig. 6 is an end view of the stent of Fig. 5.
Fig. 7 is a perspective view of the stent of Fig. 5.
Fig. 8 is a perspective view of the stent of Fig. 5 in a compressed, elongated configuration.
Fig. 9 is a plan view of the stent of Fig. 1 in a compressed, elongated configuration and mounted over a pusher catheter for placement in the vasculature.
Fig. 10 is a plan view of the stent of Fig. 1 in a compressed, elongated configuration and mounted over a pusher catheter for placement in the vasculature.
Fig. 11 is a plan view of the stent of Fig. 1 showing the stent in its expanded configuration deployed in the vasculature, and allowing retraction of a pusher catheter.
Fig. 12 is a plan view of the stent of Fig. 5 in a compressed, elongated configuration and mounted over the pusher catheter of Fig. 9 for placement in the vasculature.
Fig. 13 is a plan view of the stent of Fig. 5 showing the stent in its expanded configuration deployed in the vasculature, and allowing retraction of a pusher catheter.

### DETAILED DESCRIPTION OF THE PREFERRED EMBODIMENTS

While stents formed from polymeric memory materials are known that transform from a compressed configuration to an expanded configuration, stents formed of shape memory polymeric materials typically do not provide adequate structural and mechanical radial strength requirements for a stent, and in the past have been formed of a shape memory polymer having a glass transition temperature (T_{g}) below body temperature, making the transition from a compressed configuration to an expanded configuration more difficult to control when the stent is exposed to body temperature.

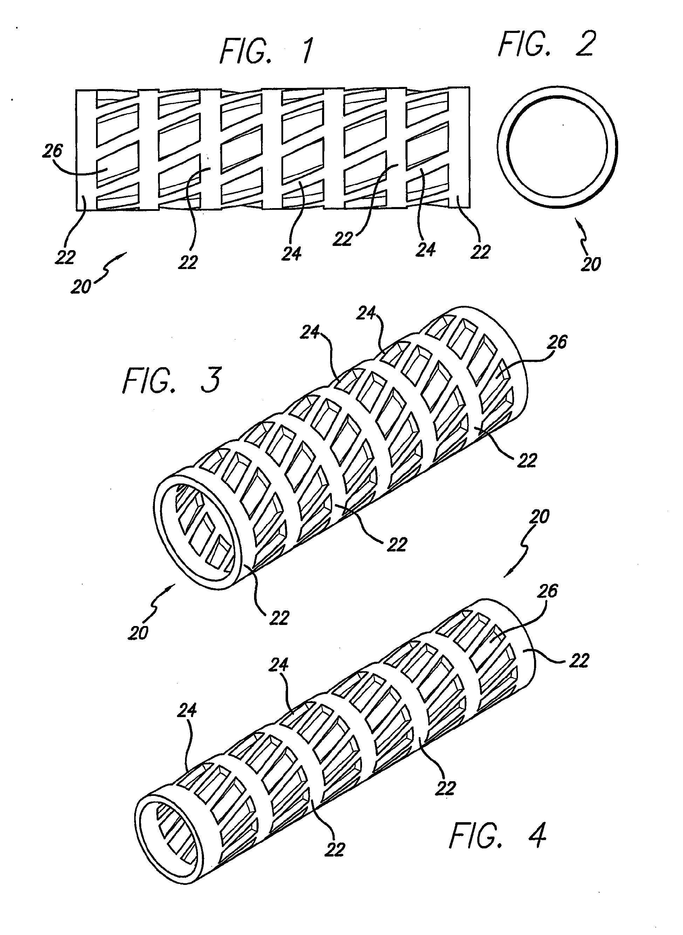

As is illustrated in the drawings, the invention is embodied in a shape memory polymer stent for treatment of a target site in a body lumen, or an intravascular flow modifier (IFM) having a tubular framework, for use in treating aneurysms such as cerebral or abdominal aneurysms. Referring to Figs. 1-4, in one presently preferred embodiment, the shape memory polymer stent or IFM 20, having a tubular framework, is preferably formed from a plurality of annular support members 22 and a plurality of cross-struts 24 intersecting the plurality of annular support members. Referring to Figs. 1 and 2, the shape memory polymer stent can be formed from an extruded tube, and can be processed to remove segments, such as by cutting a plurality of openings 26 in the extruded tube with a laser, for example, to form the intersecting annular support members and the plurality of cross-struts, providing a truss-like design. In a presently preferred embodiment, illustrated in Fig. 3, the shape memory polymer is a polyurethane that can take a predetermined shape having an expanded diameter, such as 3 mm. for example, after heating above its Tg (glass transition temperature), and a reduced diameter, shown in Fig. 4, such as of about 1 mm., for example, to fit into a catheter or delivery system. In a presently preferred embodiment illustrated in Figs. 1, 3 and 4, the cross-struts are formed to extend at an oblique angle relative to the plurality of annular support members. Alternatively, the cross-struts could be formed at other angles, such as to intersect orthogonally with the annular support members, for example.

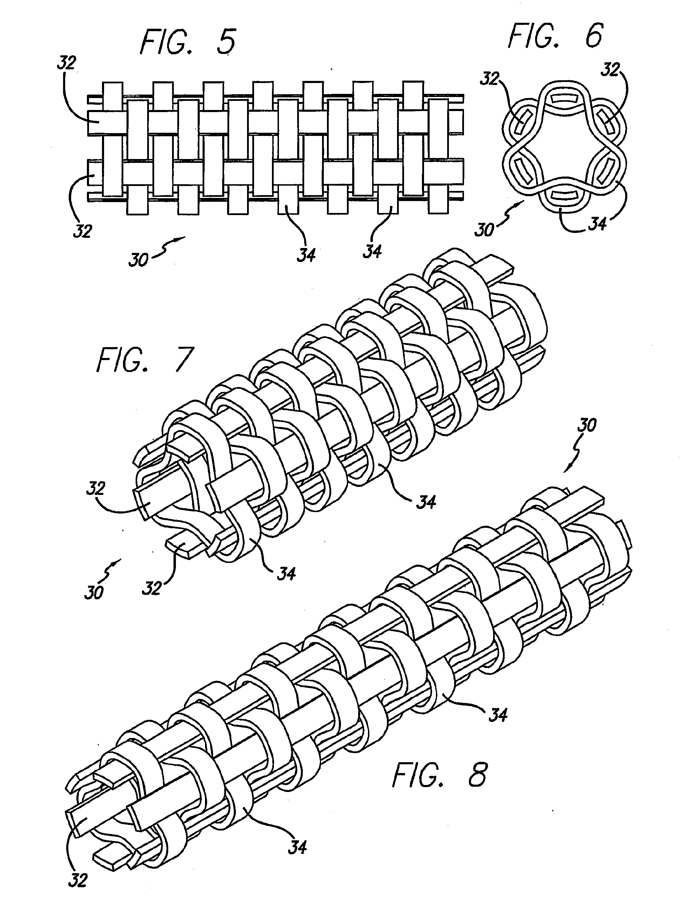

Referring to Figs. 5-8, in a second preferred embodiment, the present invention provides for a woven stent or intravascular flow modifier (IFM) 30 that can be woven from extruded strands to form a shape memory polymer stent or IFM having a tubular framework. Referring to Figs. 5 and 6, the woven shape memory polymer stent can be woven from extruded strands forming a longitudinal warp of cross-struts 32 and an annular woof of support members 34 forming orthogonally intersecting strands. Alternatively, the woven shape memory polymer stent or IFM can be woven from extruded strands forming a longitudinal warp and a spiral woof of intersecting strands. In a presently preferred embodiment, the woven shape memory polymer stent is formed from polyurethane that can take a predetermined shape, shown in Fig. 7, having an expanded diameter, such as 3 mm. for example, after heating above its Tg (glass transition temperature), and a reduced diameter, shown in Fig. 8, such as of about 1 mm. for example, to fit into a catheter or delivery system.

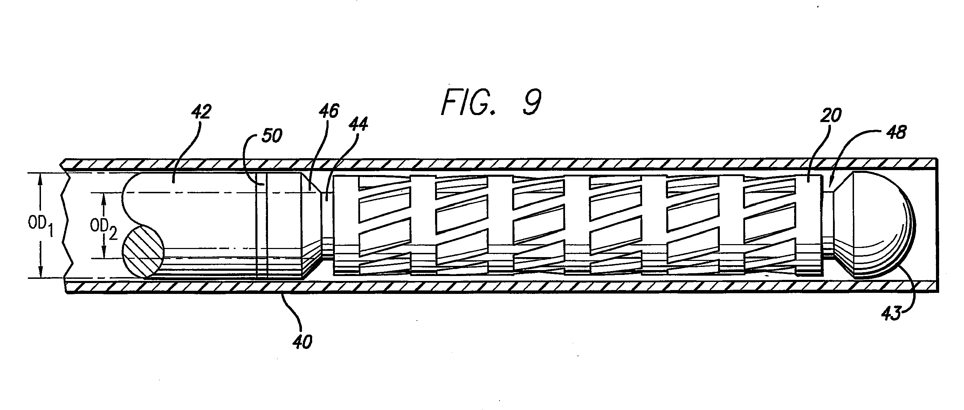

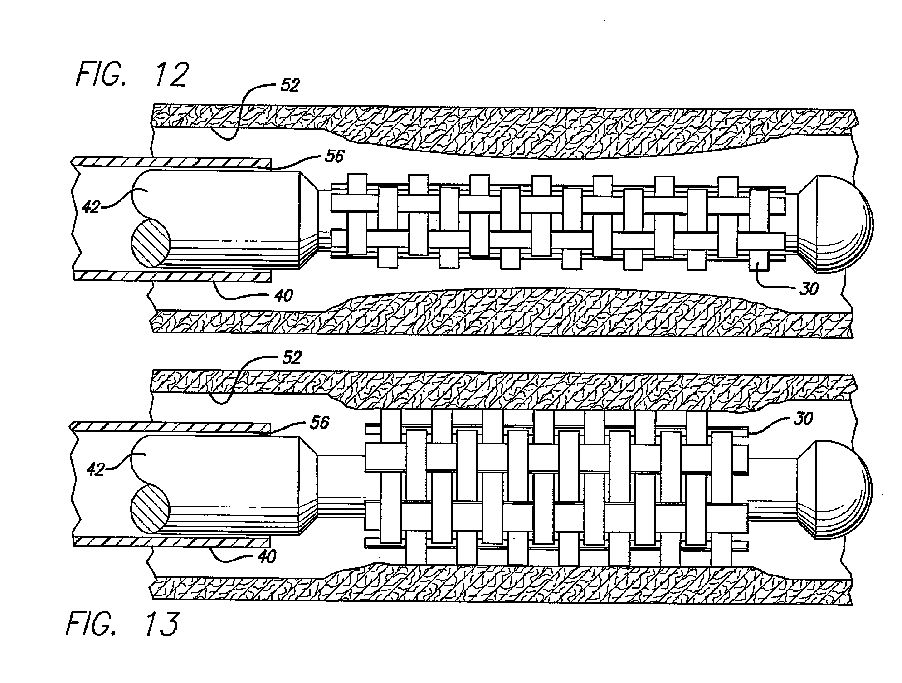

Referring to Figs. 9 and 12, in the method of the invention, the extruded shape memory polymer stent or IFM 20, or the woven shape memory polymer stent or IFM 30, can be introduced through an introducer catheter into a target site of a blood vessel to be treated in a compressed, elongated configuration, by mounting the stent over an elongated pusher catheter or pusher member 42 having a distal end 43, for placement in the vasculature. The proximal end of the pusher member is not shown, for simplicity. The pusher member can be formed from a fiber optic member, having an inner optical conductor portion 44, and an outer buffer layer 46. As is illustrated in Fig. 9, the pusher member preferably has a principal outer diameter (OD1) over the majority of the length of the elongated pusher member, and a distal region of the fiber optic member having at least a portion of outer buffer layer removed to provide a distal seating region 48 having a recessed outer diameter (OD2) that is less than the principal outer diameter, over which the shape memory polymer stent can be mounted. In a presently preferred embodiment, as is illustrated in Fig. 9, one or more radiopaque markers 50 may also be provided on the pusher member.

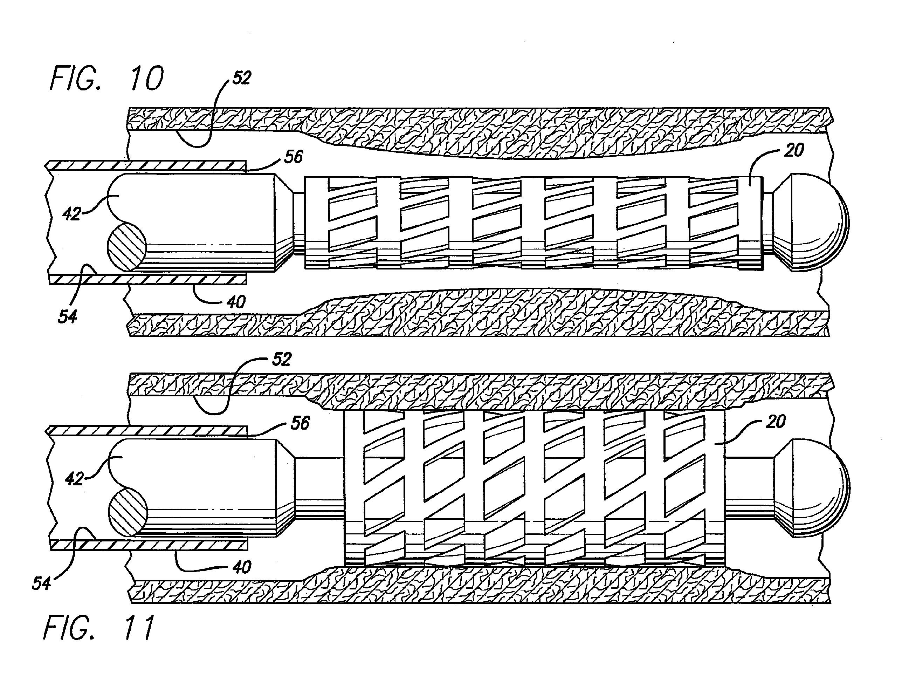

Referring to Figs. 10 and 12, in a compressed, elongated configuration mounted over a pusher member for placement in the vasculature, an extruded tubular shape memory polymer stent or IFM 20, or a woven shape memory polymer stent or IFM 30, can be placed in a body lumen such as a blood vessel 52 at a target location of a stenosis by introducing the distal seating portion of the elongated pusher member and tubular shape memory polymer stent mounted thereon into a lumen 54 of the introducer catheter, positioning the catheter within the blood vessel or other body lumen so that the distal opening of the catheter is proximal to the target site to be treated, and pushing the distal seating portion of the elongated pusher member carrying the tubular shape memory polymer stent out of the distal opening 56 of the catheter to the target site to be treated. As is illustrated in Figs. 11 and 13, the extruded or woven tubular shape memory polymer stent or IFM can be heated to cause the shape memory polymer stent or IFM to transition to the expanded configuration, thereby deploying the tubular shape memory polymer stent within the target site of the blood vessel or body lumen, or within an aneurysm and at least partially occluding the opening between the aneurysm and the parent blood vessel, and allowing retraction of a pusher member. The shape memory polymer stent or IFM can be heated by causing energy to be transmitted through the elongated pusher member to release the connection between the pusher member and the shape memory polymer stent or IFM. In a presently preferred embodiment, the pusher member comprises a fiber optic member, so that the tubular shape memory polymer stent can be heated by conducting light energy through the fiber optic member to the seating region of the elongated pusher member to heat the shape memory polymer stent or IFM. Alternatively, the elongated pusher member can be a heat pipe, and the shape memory polymer stent or IFM can be heated by conducting heat along the heat pipe elongated pusher member to the seating region of the elongated pusher member to heat the tubular shape memory polymer stent. In another alternate embodiment, the shape memory polymer stent or IFM can be heated by heating the shape memory polymer stent or IFM by conducting electricity through electrical resistance, transmission of radio-frequency electro-magnetic waves (RF) or ultra-sonic waves, or other similar means.

It will be apparent from the foregoing that while particular forms of the invention have been illustrated and described, various modifications can be made without departing from the spirit and scope of the invention. Accordingly, it is not intended that the invention be limited, except as by the appended claims.

## Claims

1. An intravascular flow modifier, comprising:
a plurality of annular support members; and
a plurality of cross-struts, said plurality of annular support members being interwoven with said plurality of cross-struts to form a tubular framework of said intravascular flow modifier, said plurality of annular support members and said plurality of cross-struts being formed of a shape memory polymer having shape memory properties and having a glass transition temperature which is above body temperature,
wherein said tubular framework has a compressed configuration at a temperature below body temperature and an expanded configuration at a temperature above body temperature, the tubular framework in the expanded configuration having an enlarged inner and outer diameter when heated above the glass transition temperature.

2. The intravascular flow modifier of Claim 1, wherein said plurality of cross-struts extend longitudinally.

3. The intravascular flow modifier of Claim 1, wherein said shape memory polymer is polyurethane.

4. The intravascular flow modifier of Claim 1, wherein when said tubular framework is in said compressed configuration said reduced outer diameter of said tubular framework is configured to fit into a corresponding catheter or delivery system.

5. The intravascular flow modifier of Claim 1, wherein when said tubular framework is in said expanded configuration the reduced inner diameter is smaller than a principal outer diameter of an elongated pusher member and larger than an outer diameter of a seating region of the elongated pusher member.
